# EUROPEAN PATENT APPLICATION

(11) **EP 3 903 667 A1**
(43) Date of publication of application: **03.11.2021**
(21) Application number: 19906493.2
(22) Date of filing: 26.12.2019
(51) Int. Cl.: A61B 5/00, A61B 5/103, A61B 5/16, A61B 5/04, A61B 5/05, A61B 5/055, A61B 8/08

(54) **SYSTEM AND METHOD FOR THE TREATMENT OF ADDICTIONS OF AN INDIVIDUAL IN NEED THEREOF, WITH LOW RELAPSE RATES**

(30) Priority: 27.12.2018 CL 20183843
(71) Applicant: Pontificia Universidad Católica De Chile, 8331150 Santiago (CL)
(72) Inventor: SITARAM, Ranganatha, Condominio Puente Piedra, Casa TH1 Santiago (CL); RUIZ POBLETE, Sergio Marcelo, 121 Santiago (CL); RANA, Mohit, Tübingen (DE)
(74) Representative: Zehetner Patentanwälte
(86) International application number: PCT/CL2019/050151
(87) International publication number: WO 2020/132769

(57) **Abstract**

The invention relates to a system, in particular an NFB system, and method which permits the treatment of addictions, of the types involving the insular cortex, of an individual in need thereof, with low relapse rates.

## Description

### TECHNICAL FIELD

The present invention falls in the field of medical addiction treatment.

### BACKGROUND

The present invention attempts to suppress proprioceptive information of craving to reach the conscious awareness of an addict. This interoceptive effect is associated with all drugs of abuse even though different drugs impart distinct subject's qualities to drug-use ritual. Several studies have reported involvement of insula in the conscious interoceptive effect and in the subjective feeling of conscious urges of all drugs, e.g., Cigarettes, Cocaine, Alcohol or Heroin. Thus, the present invention is directed to solve the problem of addiction wherein the insula, in conscious interoceptive effects, is involved. In order to provide a concrete example of how the present invention works, all descriptions found in the present document are directed to a specific example which corresponds to the treatment of nicotine addiction. Nevertheless, this should not be understood as a limitation of the present invention to this particular case: as it was previously indicated, the invention is suitable to be used in addictions wherein insula is involved.

Addiction to nicotine is one of the most common addictions in the world and one of the leading causes of preventable illness and death, worldwide. The recognized basic factors leading to nicotine addiction are social, environmental, psychological, and genetic. Around 45 million people are addicted to cigarette smoking only in the USA.

Most smokers (>70%) want to quit, and 40% attempt to do so each year; yet, only about 5% are successful without any intervention. Globally, more than six million deaths each year are attributed to tobacco use, with the accumulated loss of life expected to reach one billion by the end of the 21st century.

Drug addiction establishes due to several adaptations within the brain, which promote increasing amounts of drug use, difficulties to quit, and relapse, even though addicts are aware of all the negative consequences. There are a few cessation interventions available nowadays, i.e cognitive behavioral therapy (CBT), nicotine replacement therapy (NRT), or pharmacological therapy. Despite current treatments for nicotine addiction, relapse rates within 6 months remains high (75-80%).

Current approaches to aid people addicted to cigarette smoking include professional group therapy or counseling. These approaches achieve an initial cessation rate of 60-100% and a 1-year cessation rate of approximately 20%. A typical cessation program consists of cognitive behavioral therapy (CBT), nicotine replacement therapy (NRT) or a combination of both. CBT is a psychological intervention that helps people in changing and restructuring their thought processes, combined with acquiring new learning behaviors. It is targeted to people who want to quit smoking and maintain cessation. CBT consists of bi-weekly meetings, which are provided usually for 8-12 weeks. Increasing the length of CBT does not result in better abstinence rates.

On the other hand, NRT reduces withdrawal symptoms in smokers by replacing a small part of the nicotine usually provided by cigarettes. However, the process of nicotine delivered by NRT is different from the usual way of nicotine delivery (i.e., smoking a cigarette) both by the way it is administered and the rate at which nicotine is delivered to the brain. Consequently, NRT can reduce, but not necessarily eliminate, craving and/or withdrawal symptoms as compared to placebo. Thus, a combination of CBT and other cessation strategies like NRT and oral therapy have higher successful rates. However, neither CBT and NRT target cue-induced craving, which results in low abstinence rate over a long periods of time.

There are two types of urges that smokers face while in abstinence from nicotine, namely, withdrawal and cue-induced urges. Smokers experience withdrawal urges predominantly during the early stages of abstinence and they last for a few weeks. On the other hand, cue-induced urges are persistent over a long period and can be triggered even after years of abstinence. CBT helps smokers to develop effective coping skills for withdrawal urges by anticipating possible problems and enhancing patients' self-control. Similarly, other methods like nicotine replacement or other pharmacological therapies also help patients to cope with withdrawal urges by replacing the drug. However, none of the above-mentioned interventions targets the cue-induced craving which persists even after months of abstinence. Cue-induced urges are more likely to play a major role in relapse after a sustained period of abstinence.

In the present invention, the following definitions are considered:
Neurofeedback(NFB): is an operant conditioning procedure by which humans (or animals) can learn to modulate neural activity in one or more brain regions. For example, with the help of neurofeedback training using real-time fMRI (rtfMRI) volitional increase or decrease of Blood Oxygenation Level Dependent (BOLD) response in a circumscribed brain area or in a neural network of brain regions can be achieved. This learned volitional control over brain activity has been shown to lead to behavioral modifications
fMRI: functional magnetic resonance imaging is an indirect way to evaluate brain activity by assessing the change in blood flow in different brain regions associated with stimulus presentation.

Blood Oxygenation Level Dependent (BOLD): is a unitless number that provides an indication of a change in the blood flow to different part of the brain. During NFB training, this BOLD signal will be extracted and processed in real-time and will be linked with the reward presented to the participant based on the level of self-regulation that the participant achieves. This type of reinforcement learning allows smokers to alter their brain activity and their craving behavior.

Currently, all cessation programs work on the physical aspect of the addiction, i.e., how to enhance coping skills of the smokers for the withdrawal symptoms. Most of the withdrawal symptoms wear off by the first few weeks of abstinence. Thus, smokers are unable to efficiently target the cue-induced craving, which is the main reason for the relapse.

The present invention's new approach targets the cue-induced craving even before smokers reach conscious awareness of their craving. Another important factor is that participants are trained based on instrumental learning thus increases the success rate of the intervention as the smokers do not need to make a conscious effort to train their brain to avoid the habitual response towards smoking cues. In addition, this intervention reduces the "performance stress" for the smokers who repeated failed to quit smoking.

Neurofeedback studies on nicotine addiction have shown behavioral changes after the neurofeedback training. Unlike our invention, none of the previous studies has been directed to tackle cue-induced urges before they come to a conscious awareness of the smokers.

The present invention corresponds to a system that combines a brain-computer interface (BCI) and a neurofeedback (NFB) paradigm that can be used for reducing nicotine addiction, and to avoid relapsing. The invention also considers the treatment of nicotine addiction using the previously mentioned system. In the present document, the neurofeedback paradigm system will be referred to as the NFB system.

The inventors performed a prior art search, looking for similar technologies, and found some documents mentioning some concepts present in the instant invention, nevertheless, none of them describe all the technical details and advantages that the present invention has. In the following lines there is a summary of the documents found in such prior art search:
US2016267809A1 describes a system for brain exercise training using neurofeedback techniques. In the specification, there is a mention (paragraph 131 and 132) focused on the use of the system for treating addiction related conditions, mentioning among many others, nicotine addiction.
US2007250119A1 describes systems and methods for management of brain and body functions and sensory perception as well as systems and methods for treating diseases and conditions, among other uses. US2015290453A1 and US2009306741A1 are similar to US2007250119A1, but the claims are directed to describe a tongue-activated device.
WO2013162295A1 describes a system and method for modulating brainwaves by controlling the volume of sound. The invention is described as including a brainwave sensor that depending on the output desired, the system is configured to activate different sounds.
WO2012117343A1 describes a device and method for cognitive enhancement of a user. The specification describes that the method is based in a neurofeedback treatment, and the device corresponds to a light emitting device configured to provide light specific frequencies for stimulation of the user.
WO2010053976A2 describes the use of a neurofeedback technique combined with the administration of a drug that would enhance the response to the neurofeedback treatment. In the specification is mentioned that this technique combining neurofeedback and a specific class of drugs can be used for controlling activation of the amygdala, and that this brain region is considered to be overactive in different disorders, among them, addictions.
US2006281543A1 describes a wagering game machine. In the specification, there is a mention to the use of biofeedback or neurofeedback, wherein one of the mentioned uses is nicotine addiction treatment. The machine described in the claims does not include such feature, although it describes that a neurofeedback is included to provide a mood enhancing effect.

Thus, there is a need for an effective therapeutic intervention as more than 80% of patients who attempt to quit smoking relapse within 1 year of abstinence.

The present invention is a NFB system and method that results in an efficient approach to target the issue of high relapse rate among cigarette smokers, by training specific brain areas related to cue-induced craving, with a minimal conscious effort from smokers.

### BRIEF DESCRIPTION OF FIGURES

Figure 1. Overview of an rtfMRI-based neurofeedback system that comprises the following subsystems: (A) Participant in the MRI scanner. (B) Signal acquisition (fMRI data) using an EPI pulse sequence. (C) Online analysis and computation of the neurofeedback based on the BOLD response (D) Visual feedback via the scanner projection system. This figure is adapted from (Birbaumer et al. 2013). The rtfMRI system presented in this figure was developed at the Laboratory for Brain Machine Interfaces and Neuromodulation, Pontificia Universidad Catolica de Chile, Santiago, Chile.

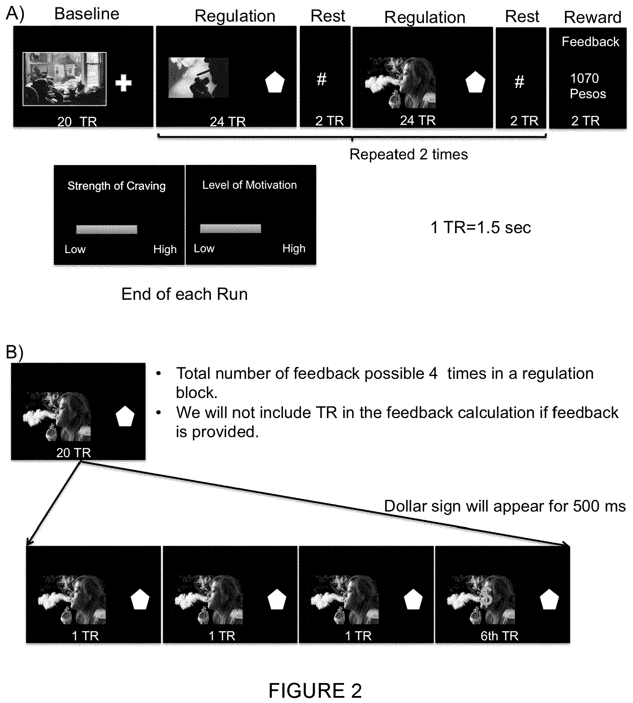

Figure 2: A) Neurofeedback run consists of one baseline (30 secs) and three regulation blocks (36 secs). During both blocks, participants will be presented with craving eliciting images and they will be differentiated by a "+" an " 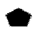 " sign, respectively. At the end of each block, reward will be presented to the patients. Also, patients need to respond to questions related to craving and motivation levels. B) During the regulation block, the patients will be briefly presented with a dollar sign whenever the BOLD signal is down-regulated during the regulation block as compared to the baseline block. Patients will press a button whenever they see the dollar sign.

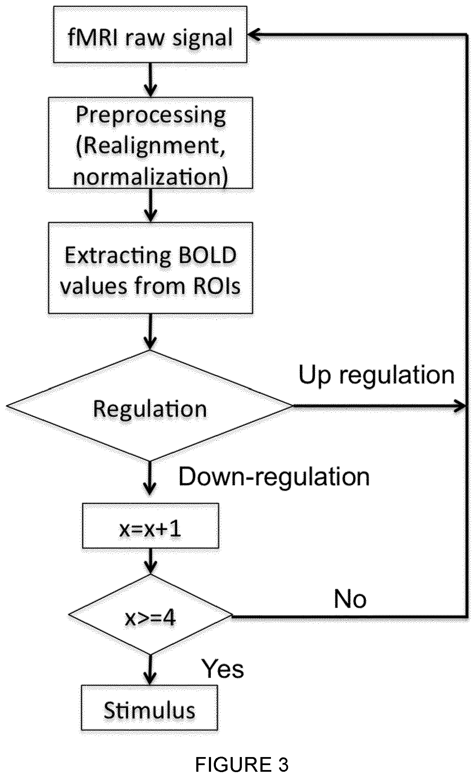

Figure 3: Block diagram showing steps to calculate feedback.
Figure 4: Interface section of the Graphic User Interface (GUI) used to specify the information related to the participant.
Figure 5: Interface section of the Graphic User Interface (GUI) used to determine the frequency at which neurofeedback will be to participants, whether to increase or decrease activity in a part of a brain region or to provide feedback based on the connectivity of two brain regions.
Figure 6: Interface section of the Graphic User Interface (GUI) used to specify which particular part of a protocol is running. For example: anatomical run, dummy functional run, or feedback run.
Figure 7: Real-time pre-processing steps to bring subject-specific data to MNI coordinates.
Figure 8: Block diagram showing mechanism for true baseline calculation.
Figure 9: Average percentage for each neurofeedback run for all the 6 fMRI sessions. Higher negative value of percentage BOLD change indicates higher downregulation during the regulation block as compared to the moving baseline as explained above. Significantly lower percentage change of the BOLD signal in the bilateral insula (A) and left insula (B) in experimental group as compared to the control group. However, no significant difference was observed in case of right insula(C).
Figure 10: Questionnaire of smoking urges-brief (QSU-b) scores in the before and after the neurofeedback training in the experimental and the control group. A significant decrease in QSU-b score is observed in the experimental group the before training session as compared to the control group. This result is suggesting that participants in the experimental group were not feeling craved to smoke cigarettes before the start training, which is in line with the VAS-C score.
Figure 13: Visual analogue scale for craving (VAS-C) scores in the before and after the neurofeedback training in the experimental and the control group. A significant decrease in VAS score is observed in the experimental group the before training session as compared to the control group. This result is suggesting that with the training participants in the experimental group were not feeling craved to smoke cigarettes before the start training.

### SUMMARY OF THE INVENTION

The present invention corresponds to a system, more particularly a NFB system, and method, that allows treatment of addictions, wherein the insula is involved, of an individual in need thereof, with low relapse rates.

### DETAILED DESCRIPTION OF THE INVENTION

As previously mentioned, the present invention corresponds to a system, more particularly a BCI-NFB system, and method, that allows treatment of nicotine addiction of an individual in need thereof, with low relapse rates.

In a first aspect, the system of the invention comprises the following components:
i. a brain-computer interface (BCI)system , capable of determining volitional increase or decrease of Blood Oxygenation Level Dependent (BOLD) response in a circumscribed brain area or in a neural network of brain regions in real-time, generating a first data set;
ii. a processing device, capable of analyzing, in real-time, the first data set generated with the BCI, and producing a second data set;
iii. a presenting device, capable of presenting feedback information based on the second data set obtained with the processing device.

In a particular embodiment of the BCI-NFB system of the invention, the circumscribed brain area is the bilateral insular cortex.

In a different embodiment, the brain signal acquisition device is a fMRI scanner.

In another embodiment, the processing device performs procedures with the data in the k-space, such as distortion correction, averaging of the signal and image reconstruction. More specifically, the procedures are performed by the same processing device.

In a more specific embodiment, said procedures are performed on the scanner's image reconstruction computer.

In a further embodiment, the processing device also processes the reconstructed image obtained from an image reconstruction computer and applies different processing techniques including realignment step to perform 3D motion correction and normalization step to map the fMRI data from a subject space to standard brain space.

In another embodiment, the processing device allows to define regions of interest (ROI) of specific size (in mm) and shape (Cube or Sphere). The position of ROI is defined by using Montreal Neurological Institute (MNI) coordinates specific to the brain region. In a further embodiment, the BOLD values pertaining to these ROIs are processed in order to calculate the feedback, which is then presented in the presenting device, previously described.

When in operation, the system forms a neurofeedback close loop, using the BOLD signals from one circumscribed brain region or circumscribed network of regions, in real-time, to calculate and present the feedback in the presenting device.

In a specific embodiment, the presenting device allows the presentation of different types of feedback. In a particular embodiment, the types of feedback are selected from a visual feedback, or an auditory feedback.

In a very specific embodiment, the BCI interface corresponds to a magnetic resonance imagining (MRI) device. In a more specific embodiment, the BCI interface corresponds to a functional MRI (fMRI) scanner.

In a second aspect of the present invention, the method to operate the system of the invention is considered. The method consists in using the NFB system to train an individual in need thereof to increase or decrease his/her brain activity of a circumscribed brain region using brain activity information, in real-time, from the brain region without conscious awareness of the mental strategy to be learned nor that they are learning brain self-regulation.

In a specific embodiment, the method considers the following steps:
1. selecting an individual with an addiction wherein the insula is involved;
2. subjecting the individual to a brain scan, using the BCI described in the system of the present invention, while presenting to the individual crave-inducing images;
3. obtaining data from a circumscribed brain area or in a neural network of brain regions of the individual, in real-time, generating a first data set;
4. processing said first data set in a processing device, and producing a second data set;
5. presenting a feedback to the individual based on the nature of the second data set;
6. iterate from step ii).

In this method, the individuals are not aware that they are controlling their brain activity.

In a specific embodiment, the individual is presented craving eliciting images, randomly appearing feedback cues (e.g., smiling faces or dollar sign), and monetary reward based on the brain activity change.

In a particular embodiment, the feedback cues are a type of implicitly presented feedback that the participants receive when they achieve a specific pattern of brain activity. Thus, this method uses a form of Instrumental Learning, in which the brain makes a correlation between a specific brain pattern and the dollar sign without conscious awareness of the participant.

To avoid performance related issues, the method of the invention provides participants with disguised information regarding the task and the feedback will be provided to them as a random event in the whole neurofeedback run. Thus, the individual will not be consciously aware of being performing a neurofeedback task, which will reduce performance pressure on the individual.

Also, as the individual is not consciously aware about the neurofeedback task, the cognitive load related to dual-task (i.e., participants have to perform to find cognitive strategy to get feedback and simultaneously they have to evaluate the feedback presented to them) would be less.

Thus, in the method of the present invention, individual will be instructed that this task is to study brain areas related to addiction, attention and processing of reward.

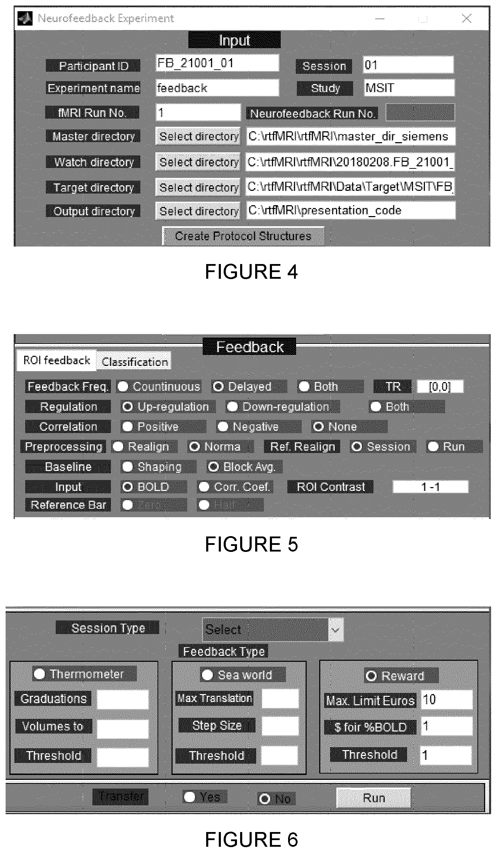

In addition, their task is to observe craving eliciting images and press button in response to certain stimuli. During the whole run, participants will be asked to observe different craving eliciting images presented to them, which will be divided between baseline blocks and regulation blocks (Figure 4). However, this information about different blocks will not be provided to the participants.

During the regulation block, dollar sign (stimulus) will appear randomly on the screen for 500 ms. This stimulus will appear if patient's brain activity in the posterior insula as a region of interest (ROI) is lower than the baseline brain activity (feeling craved). Participants will be instructed to press the button when they see the stimulus. To reinforce learning, reward will be provided to the participant at the end of each run based on their self-regulation performance.

### DESCRIPTION OF EMBODIMENTS

### EXAMPLE 1: Real-time functional magnetic resonance imaging (fMRI) based BCI-Neurofeedback system

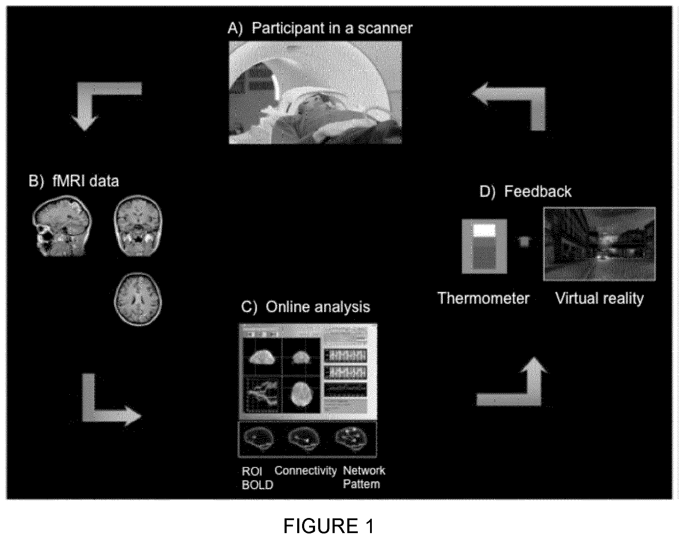

As depicted in Figure 1, the rtfMRI neurofeedback system is a close loop system that uses the BOLD signal from one circumscribed brain region or circumscribed network of regions, in real-time, to calculate and present (e.g., visual, auditory, or tactile) feedback to a participant. The rtfMRI system consists of the following subsystems: (1) Individual, (2) signal acquisition, (3) online signal analysis, and (4) feedback (see Figure 1).

An echo planar imaging (EPI) sequence is used to acquire the brain signal. Procedures with the data in the k-space such as distortion correction, averaging of the signal and image reconstruction are performed on the scanner's image reconstruction computer. Once the image is reconstructed and pre-processed, it is exported to the signal analysis subsystem. The signal analysis subsystem is implemented using an in-house Matlab (Mathworks, Natwick, MA) toolbox that retrieves the reconstructed images performs preprocessing steps like realignment and normalization. This toolbox allows to users to define ROIs of specific shape and size at the specific brain region defined by MNI coordinates. The average BOLD values pertaining to these ROIs are used to calculate the feedback, which is then presented to the participant inside the scanner.

### EXAMPLE 2: Development of real-time functional magnetic resonance imaging toolbox.

The main motivation for creating this toolbox was to provide a simple user interface to perform real-time single region of interest, connectivity of two brain regions or multivariate analysis to compute neurofeedback using fMRI signals for use in clinical rehabilitation as well as in neuroscience research. Another advantage of this toolbox is that it brings subject specific fMRI data to a standard MNI space. Due to this, extracting the data from a specific brain region across the subject does not require any input from the experimenter. Thus, it removes the bias in drawing the ROIs based on the activation pattern. For example, in Turbo BrainVoyeger (TBV) experimenter must select ROIs based on activation patterns generated by software using real-time general linear model (GLM).

The Toolbox also encompasses a graphic user interface that allows setting of specific parameters, and also for modulating feedback signals, as well as providing indications as to what protocol for data collection/production will be used, for example, an anatomical run, a dummy functional run, or a feedback run.

The interface shown in Figure 6 is used to specify the information related to the participant: like participant ID, the number of times participants have come for the neurofeedback training (session) and the number neurofeedback run that we are running (Neurofeedback Run no.). This option allows to pseudo-randomized stimulus presentation, e.g., smoking-related images. The information related to directories is required to determine where the toolbox need to look for protocol information, the upcoming MR data, folder to communicate with other stimulus presentation software and where to save preprocessed data.

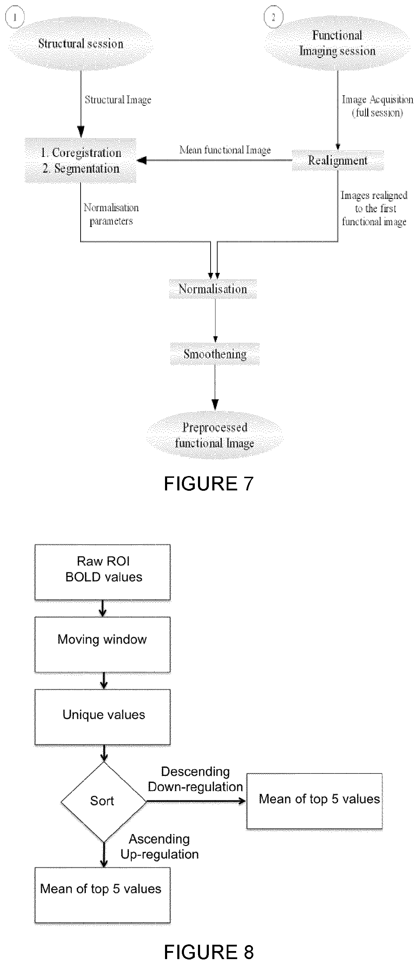

The interface shown in Figure 7, helps the code to determine the frequency at which neurofeedback will be to participants, whether to increase or decrease activity in a part of a brain region or to provide feedback based on the connectivity of two brain regions. An experimenter can also choose the preprocessing steps that they want to include in the pipeline of computing feedback. Another option allows the experimenter to choose different input data for computing feedback, e.g., whether use only BOLD values as an input to compute feedback or a correlation of BOLD values for two brain regions. The experimenter can also specify a reference area. A reference area is a task-unrelated reference ROI (e.g., auditory cortex for a motor task). The reference area is used to subtract the global (whole-brain) increase in the BOLD signal due to general arousal, task-unrelated factors, or BOLD fluctuations caused by head motion.

The interface shown in Figure 8, experimenters need to specify which particular part of the protocol they are running: for example anatomical run, dummy functional run, or feedback run. Additionally, experimenters have multiple options for presenting feedback to participants. Either it can be a simple thermometer wherein the number of bars represents the level of their brain activity or virtual reality-based feedback where they can change the different aspect of the scenario based on the activity in different regions of the brain. Another possibility is to provide reinforcement to the participants along with the visual feedback in the form of money. Real-time pre-processing steps to bring subject-specific data to MNI coordinates is achieved in the following steps: 1) An Anatomical scan and a dummy functional scan were acquired. The dummy functional run comprises of 30 T2* images acquired using EPI sequence. These functional images were realigned to obtain a mean functional image, which is necessary for the co-registration process.

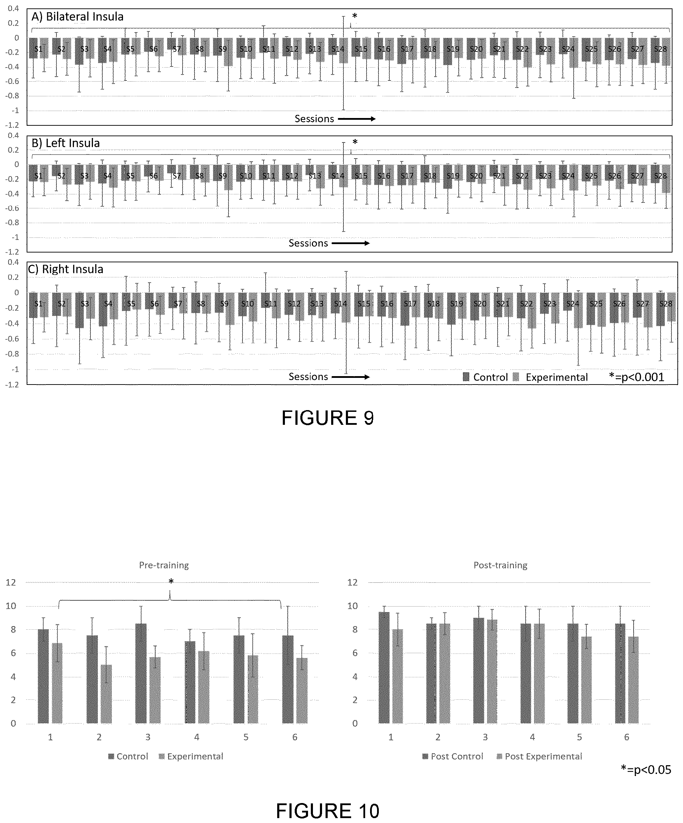

Figure 9 shows a block diagram showing different steps of the protocol:
1. Co-registration process finds the transformation matrix that maps the anatomical image into the space of the functional images specified by the mean functional image created in the previous step.
2. Segmentation process classifies the voxels of an anatomical image into three classes of white matter, grey matter, and cerebrospinal fluid. It computes the affine transformation matrix that is later used to normalize the upcoming T2* images into standard MNI brain space.

The first steps were prerequisite to calculate parameter to alienate inter-subjects. The following steps were performed, in real-time, in order to compute feedback:
1. Functional data, pertaining to the task, is converted from the DICOM format into the ANALYZE format (*.hdr, *.img) used in SPM toolbox.
2. Realignment of raw functional scan is performed to the first T2 image acquired during the dummy functional run (step 1), followed by the
3. normalization using the parameters previously calculated.
4. Once data is normalized, the ROI is built around the MNI coordinate that experimenter provides in the configuration file of the toolbox.

### Development of a new mechanism to calculate true baseline (Fig. 10):

Traditionally, neurofeedback training is composed of alternating blocks of baseline and regulation blocks. The feedback is calculated as the change in BOLD values during the regulation block as compared to the average BOLD values during baseline block. However, this is not the best way to calculate up- or down-regulation as the baseline BOLD values are calculated as mean values, but it does not include any information related to the generalized fluctuation of the BOLD signal during the baseline blocks. Thus, to achieve true up-regulation/ down-regulation the participants must increase BOLD values above the mean BOLD value during the baseline blocks plus/minus the standard deviation of the baseline blocks. Thus, we planned to use a moving averaging method to calculate the baseline level of BOLD values after each TR of the regulation block. This method will allow us to implement a shaping method introduced by B.F. Skinner. Shaping is a conditioning paradigm used primarily in the experimental analysis of behavior. In shaping, small changes towards the desired behavior are rewarded, which leads to gradual change across the successive trials. The algorithm allows the experimenter to choose a moving window of data points, e.g., 10, and then the data points from this moving window were chosen by sorted unique values in descending or ascending order depending on whether we are calculating down-regulation or up-regulation, respectively.

The benefit of using this method is that it considers the small fluctuations in the BOLD and update the baseline BOLD value.

### Neurofeedback protocol for nicotine addicts:

A new neurofeedback training protocol has been developed in which participants will be provided with disguised information regarding the task and the feedback will be provided to them as a random event during the whole neurofeedback training. Thus, participants will not be consciously aware of being performing a neurofeedback task, which will reduce performance pressure on the participants. In addition, as participants will not be consciously aware of the neurofeedback task, the cognitive load related to dual-task (i.e., participants have to perform to find the cognitive strategy to get feedback and simultaneously they have to evaluate the feedback presented to them) would be less. Thus, in the new protocol, participants will be instructed that this task is to study brain areas related to addiction, attention, and processing of reward. In addition, their task is to observe craving eliciting images and to press a button in response to certain stimuli. During the whole run, participants will be asked to observe different craving eliciting images presented to them, which will be divided between baseline and regulation blocks (Figure 1). However, this information about different blocks will not be provided to the participants. During the regulation blocks, a dollar sign (stimulus) will appear randomly on the screen for 500 ms. This stimulus will appear if patient's brain activity in the insula as a region of interest (ROI) is lower than the baseline brain activity (feeling craved). Participants will be instructed to press the button when they see the stimulus. To reinforce learning, the reward will be provided to the participant at the end of each run based on their self-regulation performance. In this pilot data, 8 participants underwent 6 sessions of training over a period of two weeks. Each participant had a medium level of nicotine dependence (FTND score>=4) and smoked more than 10 cigarettes per day. The participants in the control group received a yokt feedback in which participants were provided with average amount of reinforcement earned by the experimental group. Similarly, participants in the control group were presented with average amount of dollar signs observed by the participants in the experimental group.

A neurofeedback run consists of one baseline (30 secs) and three regulation blocks (36 secs). During both blocks, participants will be presented with craving eliciting images and they will be differentiated by a "+" and " 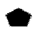 " sign, respectively. At the end of each block, the reward will be presented to the patients. Also, patients need to respond to questions related to craving and motivation levels. During the regulation blocks, the patients will be presented with a dollar sign pertaining to their brain activity.

Figure 11 shows the average percentage for each neurofeedback run for all the 6 sessions. Higher negative value of percentage BOLD change indicates higher downregulation during the regulation block as compared to the moving baseline, as explained above. Significantly lower percentage change of the BOLD signal in the bilateral insula (A) and left insula (B) in experimental group as compared to the control group. However, no significant difference was observed in right insula(C).

Figure 12 shows the results of a questionnaire of smoking urges-brief (QSU-b) scores in the before and after the neurofeedback training in the experimental and the control group. A significant decrease in QSU-b score is observed in the experimental group before the start of the neurofeedback training session as compared to the control group. This result suggests that participants in the experimental group were not feeling craved to smoke cigarettes before the start training, which is in line with the VAS-C score.

Figure 13 shows a visual analogue scale for craving (VAS-C) scores in the before and after the neurofeedback training in the experimental and the control group. A significant decrease in VAS score is observed in the experimental group before the start of neurofeedback training session as compared to the control group. This result is suggesting that with the training participants in the experimental group were not feeling craved to smoke cigarettes before the start training.

The results indicate that participants in the experimental group can learn to reduce the BOLD signal in the bilateral insular cortex in the presence of smoking cues. The participants in the control group also achieved down-regulation even though they were not provided contingent feedback. The possible reason could be the yoke feedback, wherein the participants in the control group were provided feedback randomly with the average frequency and the average amount of reward received by the participants in the experimental group. Thus, it might lead to some learning due to the higher frequency of feedback provided randomly to the participants in the control group. On the behavioral aspect of the experiment, we observe a significantly lower subjective craving scoring in the VAS-C and QSU-b questionnaires by the participants in the experimental group as compared to the participants in the control group. These two results indicate a causal relationship between the down-regulation in the bilateral insular cortex and the craving behavior. During the participant's interview at the end of the experiment, participants were asked whether they observe any specific change in their smoking behavior in general. Three participants in the experimental group mention that it was relatively easier for them to refrain themselves from smoking cigarettes before the neurofeedback sessions in the later part of the experiment. This observation coincides with the lower score in the VAS-C and QSU-b score that participant responded before the start of the neurofeedback training session. This is clinically relevant observation, as the time to smoke the first cigarette after waking up is considered as one of the main behavioral measures to determine nicotine dependence of a participant. The rtfMRI sessions were conducted around 8 am and the participants were asked to refrain from smoking cigarettes at least 3 hours before the neurofeedback session. Thus, as per the report from the participants, they smoked their last cigarettes a night before.

### INDUSTRIAL APPLICABILITY

The invention can be applied in the medical device industry.

## Claims

1. A system and method, that allows treatment of addiction, wherein the insula is involved, of an individual in need thereof, with low relapse rates, wherein the system comprises:
i. a brain-computer interface (BCI), capable of determining volitional increase or decrease of Blood Oxygenation Level Dependent (BOLD) response in a circumscribed brain area or in a neural network of brain regions in real-time, generating a first data set;
ii. a processing device, capable of analyzing, in real-time, the first data set generated with the BCI, and producing a second data set;
iii. a presenting device, capable of presenting feedback information based on the second data set obtained with the processing device.

2. System according to claim 1, wherein the system is a Neurofeedback (NFB) system.

3. System according to claim 2, wherein the circumscribed brain area is the bilateral insular cortex.

4. System according to claim 1, wherein the processing device also processes the reconstructed image obtained from an image reconstruction computer and applies different processing techniques including realignment step to perform 3D motion correction and normalization step to map the fMRI data from a subject space to standard brain space.

5. System according to claim 1, wherein the processing device allows to define regions of interest (ROI) of specific size (in mm) and shape (Cube or Sphere).

6. System according to claim 1, wherein the position of ROI is defined by using Montreal Neurological Institute (MNI) coordinates specific to the brain region.

7. System according to claim 5 or 6, wherein the Blood Oxygenation Level Dependent (BOLD) values pertaining to these ROIs are processed in order to calculate the feedback, which is then presented in the presenting device, previously described.

8. System according to claim 7, wherein the system forms a neurofeedback close loop, using BOLD signals from one circumscribed brain region or circumscribed network of regions, in real-time, to calculate and present the feedback in the presenting device.

9. In a specific embodiment, the presenting device allows the presentation of different types of feedback. In a particular embodiment, the types of feedback are selected from a visual feedback, or an auditory feedback.

10. System according to claim 1, wherein the BCI interface corresponds to a magnetic resonance imagining (MRI) device.

11. System according to claim 10, wherein the BCI interface corresponds to a functional MRI (fMRI) scanner.

12. Method to operate the system described in the previous claims, the method consisting in using the BCI-NFB system to train an individual in need thereof to increase or decrease his/her brain activity of a circumscribed brain region using brain activity information, in real-time, from the brain region without conscious awareness of the mental strategy to be learned nor that they are learning brain self-regulation.

13. Method according to claim 12, the method comprising the following steps:
i. selecting an individual with an addiction wherein the insula is involved;
ii. subjecting the individual to a brain scan, using the BCI described in the system of the present invention, while presenting to the individual crave-inducing images;
iii. obtaining data from a circumscribed brain area or in a neural network of brain regions of the individual, in real-time, generating a first data set;
iv. processing said first data set in a processing device, and producing a second data set;
v. presenting a feedback to the individual based on the nature of the second data set;
vi. iterate from step ii),
wherein the individuals are not aware that they are controlling their brain activity.

14. Method according to claim 12, the individual is presented craving eliciting images, randomly appearing feedback cues (e.g., smiling faces or dollar sign), and monetary reward based on the brain activity change.

15. Method according to claim 14, the feedback cues are a type of implicitly presented feedback that the participants receive when they achieve a specific pattern of brain activity.

16. Method according to claim 12, in order to avoid performance related issues, the method provides participants with disguised information regarding the task and the feedback will be provided to them as a random event in the whole neurofeedback run.
